# EUROPEAN PATENT SPECIFICATION

(11) **EP 1 366 409 B1**
(45) Date of publication and mention of the grant of the patent: **27.07.2011**
(21) Application number: 02733785.6
(22) Date of filing: 25.01.2002
(51) Int. Cl.: G06F 1/00, G01N 33/50

(54) **LIGHT SCATTERING DETERMINATION OF TREATMENT POTENCIES**
LICHTSTREUBESTIMMUNG VON BEHANDLUNGSPOTENZEN
DETERMINATION D'ACTIVITES THERAPEUTIQUES PAR DIFFUSION DE LA LUMIERE

(30) Priority: 01.02.2001 US 265761 P
(43) Date of publication of application: 03.12.2003
(73) Proprietor: SpectraDigital Corporation, Guelph, ON N1L 1H7 (CA)
(72) Inventor: DESHPANDE, Satish, Guelph, Ontario N1H 383 (CA)
(74) Representative: Powell, Stephen David
(86) International application number: PCT/US2002/002132
(87) International publication number: WO 2002/077748

(56) References cited:
- WO-A-2006/107718
- US-A- 4 101 383
- US-A- 4 541 719
- US-A1- 4 467 032
- US-A1- 5 726 030

## Description

This application claims benefit of US provisional application 60/265,761 filed 01 1 February 2001.
US 4,101,383 discloses a differential light scattering photometer for testing samples of bacteria in suspension. A plurality of cuvettes containing the samples is placed in a cuvette holder or carousel which rotatably places each cuvette in position to interrupt a laser beam. A detector rotates in an arc about each sample to produce output signals representing the scattered light intensity as a function of angular location, relative to the incident laser beam, about the sample. A control differential scattering pattern is produced and compared with test differential scattering patterns derived from test samples having added antibiotics. The patterns are compared by data processing to produce an output punched card indicative of bacterial sensitivity to each antibiotic. The data processor is responsive, through pattern comparison, to various responses of the bacteria, i.e., whether the antibiotic is bactericidal, bacteriostatic or the bacteria actually metabolize the antibiotic. A protocol and process is also disclosed
US 4,467,032 discloses a process and apparatus for identifying and enumerating microorganisms. A sample believed to contain a certain microorganism is combined with an inhibiting reagent having a known specificity for the microorganism. The combined sample and inhibiting reagent is placed in an alternating current electrical field having a frequency sufficiently high to cause alignment of microbial cells in the sample. A polarized beam of laser light is then passed through the sample and the extent of birefringence is measured. The measured birefringence is compared with that of a control sample known to contain the microorganism under investigation to determine the microorganism in the sample.
US 5,726,030 discloses a method of automatically testing the sensitivity of a paraffinophilic microorganism to different antimicrobial agents and concentrations thereof that includes a plurality of receptacles each containing an amount of liquid medium, an antimicrobial agent to be tested and the microorganism to be tested. A paraffin containing slide is placed in each receptacle after which incubation is effected. A light scatter sensor is employed automatically and sequentially to monitor the extent of microorganism growth on the slides and the growth on each slide is employed to determine which antimicrobial agent and which concentration is effective to resist growth of the paraffinophilic microorganism. The slides are moved sequentially in operative relationship with the light scatter sensor while resisting rotational movement of the slides. Positive and negative control slides are employed as is a computer in receiving and processing the results of the slide testing. Related apparatus is also disclosed.

The invention measures potencies of treatments which cause changes in cells by measuring difference between angular intensity distributions of light scattered by treated cells and by untreated cells.

This potency measurement is based on the unexpected discovery that treatments which cause the greatest difference between angular intensity distributions of light scattered by treated cells and by untreated cells have the greatest treatment potency.

This unexpected discovery is especially useful for determining which treatment will be most potent for a specific individual against the human immunodeficiency virus.

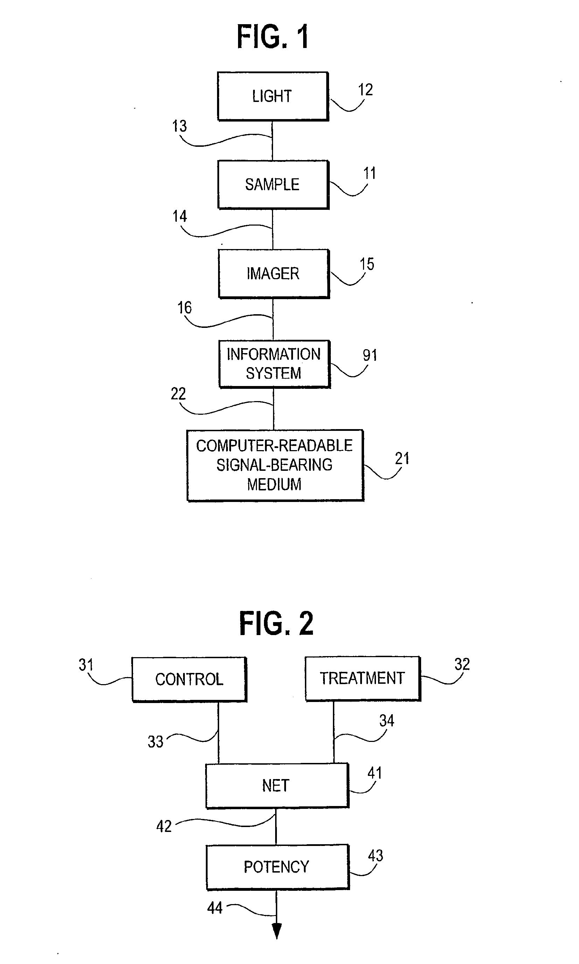

FIG. 1 schematically depicts a light scattering arrangement.

FIG. 2 schematically depicts a potency measurement.

Measuring potency of treatments on cells comprises illuminating cell samples, detecting light scattered by the samples, and causing output of a potency signal representing difference between light scattered by samples.

The cell samples comprise a control sample of cells, and a treatment sample of cells. The control sample is from a sample. The control sample can be the sample with nothing added. The control sample can be from the sample with a control treatment added. The control sample can be from the sample with a plurality of control treatments added. The treatment sample is from the sample with a treatment added. The treatment sample can be from the sample with a plurality of treatments added.

A "treatment" here - and throughout - means any substance which causes changes in parts of cells from the sample. The changes can be to surface parts of cells, to interior parts of cells, and to combinations of these. The changes can be desired changes and can be undesired changes.

The control sample 11 is illuminated 13 with control light 12. A control imager 15 detects control scattered light 14. Control scattered light is a control portion of the control light which is scattered by the control sample and which is detected by the control imager.

The treatment sample 11 is illuminated 13 with treatment light 12. A treatment imager 15 detects treatment scattered light 14. Treatment scattered light is a treatment portion of the treatment light which is scattered by the treatment sample and which is detected by the treatment imager.

The control scattered light has a control angular intensity distribution 31. The treatment scattered light has a treatment angular intensity distribution 32. A potency signal 44 which represents difference between the control angular intensity distribution and the treatment angular intensity distribution is caused by the control imager and the treatment imager.

The potency signal can represent a difference - which can be positive and can be negative - between the control signal and the treatment signal at a single scattering angle. The potency signal can represent a plurality of differences - any of which can be positive and can be negative - between the control signal and the treatment signal at a plurality of scattering angles.

Experiments show that treatments which cause the greatest potency signals have the greatest treatment potency. For example, experiments show that - for a sample prepared from a specific HIV infected blood donor - the HIV treatment which causes the greatest potency signal has the greatest potency against HIV for that blood donor.

The control light and the treatment light can be from two separate light sources, can be from the one light source, and can be from portions of one light source. The control imager and the treatment imager can be two separate imagers, can be one imager, and can be portions of one imager. An imager - and imagers, and portions of an imager - can detect the control scattered light and the treatment scattered light concurrently using beam splitting means.

The control scattered light and the treatment scattered light must be comparable. This can be achieved in various ways. For example, the conditions of the two imagings can be interchangeable, except for the added treatment, so that the control scattered light and the treatment scattered light are directly comparable. For example, the two imagings can be calibrated with a standard imaging, so that the control scattered light and the treatment scattered light are comparable via the calibrations.

Various imaging means have been used. Scattered light can illuminate a screen which can be imaged. Scattered light can be imaged directly. Useful results are obtained using commercial video cameras for the imagings. Here imaging means need not form an image. The imager can be any means for detecting an angular intensity distribution of scattered light.

A control signal 33 can be caused by the control imager. The control signal represents the control angular intensity distribution of the control scattered light. A treatment signal can be caused by the treatment imager. The treatment signal 34 represents the treatment angular intensity distribution of the treatment scattered light.

The control signal can be subtracted electronically from the treatment signal to cause the potency signal. Difference between the treatment angular intensity distribution and the control angular intensity distribution can be imaged directly using beam splitting and phase shifting means.

The control imager and treatment imager can cause the potency signal via signal connection 16 with an information system 91 and via signal connection 22 of a computer-readable signal-bearing medium 21 with the information system.

The medium can cause use of the control signal and the treatment signal to cause output of the potency signal. The medium can have a net component 41. The net component can cause calculation of a net signal 42. The net signal represents difference between the treatment angular intensity distribution and the control angular intensity distribution. The medium can have a potency component 43. The potency component can cause calculation of the potency signal 44.

The potency signal represents difference between angular intensity distributions of light scattering by treated cells and by untreated cells. These difference can be represented in various ways. For example, the area under the curve of absolute values of positive and negative intensity differences at scattering angles can be calculated. For example, the curve of values of positive and negative intensity differences at scattering angles can be normalized to make the most negative intensity difference at least zero and the area under the normalized curve calculated. Various other analytical tools can be used.

Treatments which cause changes to interior parts of cells generate intensity difference at small angles. Treatments which cause changes to surface parts of cells generate difference at larger angles. In HIV treatments studied, effects on interior parts and on exterior parts are seen in scattering angles between zero and four degrees. Effects using other treatments and other cells can be seen at scattering angles of up to about 25 degrees. The range of scattering angles which should be studied depends on the cells and treatments being examined.

The potency signal can represent intensity difference between angular intensity distributions from various parings of control samples and treatment samples. The control samples can be the sample, can be from the sample with a control treatment added, and can be from the sample with a plurality of control treatments added. The treatment sample can be from the sample with a treatment added, and can be from the sample with a plurality of treatments added. The potency signal can represent difference between angular intensity distributions from various parings of these control and treatment sample variations.

The medium can have an historical component which causes calculation of an historical signal. The historical signal represents difference between historical data and a member from a signal group consisting of the treatment signal, the control signal, and the net signal. The historical signal can have components which can represent difference between historical data and more than one member of the signal group, with each component from the components paired with a member from the signal group.

Historical data can comprise representations of historical control signals, representations of historical treatment signals, and representations of historical net signals. "Representations" can be various kinds of averages of historical data, constructions from historical data according to some criteria, bellwether data culled from historical data, and combinations of these.

Historical control, treatment, and net signals can be obtained by the means interchangeable with the means by which the control, treatment, and net signals are obtained. Historical control, treatment, and net signals can be obtained by the means comparable via calibrations with the means by which the control, treatment, and net signals are obtained.

Historical data can be used in the calculation of the potency signal. Historical data can be used in calculation of the net signal. Historical data can be used in causing the control signal and the treatment signal. Historical data can be used in configuring the imagings and in preparing the samples. In some cases historical data can be the control signal.

The historical component of the medium can be a component of the net component, can be a component of the potency component, can be a separate component of the medium, and can be combinations of these. The historical data can be part of the medium, can be available to the medium via a signal, and can be combinations of these.

A light scattering product for measuring effects of treatments on cells comprises the elements and configurations described above which obtain the potency signal. The product operates according to a light scattering method for measuring effects of treatments on cells.

The light scattering method for measuring effects of treatments on cells comprises preparing a sample of cells, preparing a control sample of cells, preparing a treatment sample of cells, illuminating the control sample with control light, illuminating the treatment sample with treatment light, detecting control scattered light with a control imager, detecting treatment scattered light with a treatment imager, and outputting a potency signal which represents difference between a treatment angular intensity distribution and a control angular intensity distribution by means equivalent to the means described above.

The light scattering method does not depend on how control and treatment samples are prepared, on how the angular scattering intensity distributions are obtained, nor on how difference between the distributions are determined. The light scattering product can use various means for preparing control and treatment sample and can use various elements and configurations to obtain the angular scattering intensity distributions and to determine difference between the distributions.

In one example, sample preparation starts with collection of about of five milliliters of blood held in an upright five milliliter tube with heparin at room temperature for about three to ten hours and then refrigerated at about four degrees Celsius until the red cells settle down and a Buffy coat is formed. The Buffy coat is removed with a Pasteur pipette and added to a tube with 10 millimoles per liter phosphate buffered saline to total typically 1.8 milliliters. This white cells solution is and kept on ice until measurements are taken.

Various other means for separating out the white cells can be used. The method and product can work without separating out the white cells.

About 1.2 milliliters of phosphate buffered saline is added to a 1.5 milliliter optical cuvette. This is assigned an optical density of zero percent. An amount of the white cells solution is added to increase the optical density by about 10 to 15 percent. This is the sample and is the control sample in this example.

The increase in optical density ensures that there are enough scattering cells to get useful scattering distributions, but not so many so that scattered photons which have been scattered by multiple cells dominate the scattered beam. The method and product can work with various cell densities and the resultant optical densities.

The cuvette with the control sample is placed between the beam of a 780 nanometer laser and a video camera imager. About twenty images at fifteen second intervals are obtained. These images are averaged using the National Institutes of Health imaging analysis package ImageJ. This is the control signal which represents the control angular intensity distribution in this example.

Other wavelengths of laser light can be used. Other methods of imaging and averaging can be used. The method and product can work with any means of detecting the angular intensity distribution of the control scattered light.

A treatment - such as amprenavir - is added to the control sample in the cuvette so that the treatment concentration in the cuvette is 100 millimoles per liter. This is the treatment sample in this example.

Incubation times of fifteen minutes to twelve hours have been used. Thirty minutes works well for human HIV treatments. The incubation time depends on the cells and treatments.

The cuvette - now with the treatment sample - is placed between the beam of the 780 nanometer laser and the imager. About 20 images at 15 second intervals are obtained. These images are averaged using the National Institutes of Health imaging analysis package ImageJ. This is the treatment signal which represents the treatment angular intensity distribution in this example.

Other methods of imaging and averaging can be used. The method and product can work with any means for detecting and quantifying the angular intensity distribution of the treatment scattered light.

The control signal is subtracted from the treatment signal pixel by pixel. Along a radius from the center of the subtracted signal, averages along a chord centered on the radius are obtained. This is the net signal representing difference between the treatment angular intensity distribution and the control angular intensity distribution in this example.

The net signal can be obtained from the control signal and the treatment signal in various ways. The potency signal can be determined from the net signal by various means. The method and product can work with various determinations of difference between the control angular intensity distribution and the treatment angular intensity distribution.

A "signal" from a product part to a second product part - and a product part being "signal connected" with a second product part - here, and throughout, mean that a physical state of the product part causes a second physical state of the second product part. This can occur by various direct causal means and can occur by any of various transmission means. Transmitted signals can be any of various point-to-point and broadcast forms of energy transmission such as wireless and via wires, cables, and fibers. Parts of transmitted signals can reside with one form of the transmitted signal, parts can reside with a second form of transmitted signal, and parts can reside with various combinations of transmitted signals.

The several causes here can act via any of various processing modes. The processing can utilize configured processing elements such as fixed circuits, can utilize configurable processing elements such as field programmable gate arrays and neural networks, can utilize instructions in a data-bearing medium, and can utilize combinations of these. The processing can be by stand alone means, can act via a local information system, can act via a networked information system, and can act via combinations of these. The processing - in part at least - can be by parts of an imager. The computer-readable signal-bearing medium can be a transmitted signal, a data storage medium, and a combination of a transmitted signal and a data storage medium.

## Claims

1. A method of studying the effect of a treatment for HIV, the method comprising:
preparing a sample of cells;
preparing a control sample of cells,
the control sample being from the sample;
preparing a treatment sample of cells,
the treatment sample being from the sample with a treatment added;
illuminating the control sample with control light;
illuminating the treatment sample with treatment light;
detecting control scattered light between 0 and 4 degrees with a control imager,
control scattered light being a control portion of the control light which is scattered by the control sample and detected by the control imager between 0 and 4 degrees,
the control scattered light detected between 0 and 4 degrees having a control, angular intensity distribution;
detecting treatment scattered light between 0 and 4 degrees with a treatment imager,
treatment scattered light being a treatment portion of the treatment light which is scattered by the treatment sample and detected by the treatment imager between 0 and 4 degrees,
the treatment scattered light detected between 0 and 4 degrees having a treatment angular intensity distribution; and
outputting a potency signal,
the potency signal representing difference between the treatment angular intensity distribution and the control angular intensity distribution, and
the potency signal being caused by the control imager and the treatment imager.

2. A method as claimed in claim 1, wherein the step of outputting the potency signal comprises:
causing a control signal by the control imager,
the control signal representing the control angular intensity distribution;
causing a treatment signal by the treatment imager,
the treatment signal representing the treatment angular intensity distribution;
using the control signal and the treatment signal to cause output of the potency signal.

3. A method as claimed in claim 2, wherein the step of outputting the potency signal comprises:
calculating a net signal;
the net signal representing difference between the control signal and the treatment signal; and
using the net signal to cause output of the potency signal.

4. A method as claimed in claim 3, including the step of causing a historical signal, the historical signal representing difference between historical data and a member from a signal group consisting of the treatment signal, the control signal, and the net signal.

5. A method as claimed in any preceding claim, wherein the method is a method of studying the effects of treatment on an interior part of a cell.

6. A method as claimed in any preceding claim, wherein the method is a method of studying the effect of a treatment on an exterior part of a cell.

7. An imaging method of studying the effect of a treatment for HIV, the method comprising:
preparing a sample of cells;
preparing a control sample of cells,
the control sample being from the sample;
preparing a treatment sample of cells,
the treatment sample being from the sample with a treatment added;
illuminating the control sample with control light;
illuminating the treatment sample with treatment light;
detecting control scattered light between 0 and 4 degrees with a control imager,
control scattered light being a control portion of the control light which is scattered by the control sample and detected by the control imager between 0 and 4 degrees,
the control scattered light detected between 0 and 4 degrees having a control angular intensity distribution;
detecting treatment scattered light between 0 and 4 degrees with a treatment imager,
treatment scattered light being a treatment portion of the treatment light which is scattered by the treatment sample and detected by the treatment imager between 0 and 4 degrees,
the treatment scattered light detected between 0 and 4 degrees having a treatment angular intensity distribution;
outputting a potency signal,
the potency signal representing difference between the treatment angular intensity distribution and the control angular intensity distribution, and
the potency signal being caused by the control imager and the treatment imager by a method comprising:
causing a control signal by the control imager,
the control signal representing the control angular intensity distribution;
causing a treatment signal by the treatment imager,
the treatment signal representing the treatment angular intensity distribution;
calculating a net signal,
the net signal representing difference between the control signal and the treatment signal and
using the net signal to cause output of the potency signal.

8. A method as claimed in claim 7, including the step of causing a historical signal, the historical signal representing difference between historical data and a member from a signal group consisting of the treatment signal, the control signal, and the net signal.

## Patentansprüche

1. Verfahren zum Untersuchen des Effekts einer Behandlung auf HIV, wobei das Verfahren aufweist:
Vorbereiten einer Probe von Zellen;
Vorbereiten einer Kontrollprobe von Zellen,
wobei die Kontrollprobe von der Probe stammt;
Vorbereiten einer Behandlungsprobe von Zellen,
wobei die Behandlungsprobe von der Probe stammt, wobei eine Behandlung hinzugefügt ist;
Beleuchten oder Bestrahlen der Kontrollprobe mit Kontrolllicht;
Beleuchten oder Bestrahlen der Behandlungsprobe mit Behandlungslicht;
Detektieren eines Kontrollstreulicht zwischen 0 und 4 Grad mit einem Kontroll-Imager,
wobei das Kontrollstreulicht ein Kontrollanteil des Kontrolllichts ist, das von der Kontrollprobe gestreut wird und durch den Kontroll-Imager zwischen 0 und 4 Grad detektiert wird,
wobei das zwischen 0 und 4 Grad detektierte Kontrollstreulicht eine Kontrollwinkelintensitätsverteilung hat;
Detektieren eines Behandlungsstreulichts zwischen 0 und 4 Grad mit einem Behandlungsimager,
wobei das Behandlungsstreulicht ein Behandlungsanteil des Behandlungslichts ist, das durch die Behandlungsprobe gestreut wird und vom Behandlungsimager zwischen 0 und 4 Grad detektiert wird,
wobei das zwischen 0 und 4 Grad detektierte Behandlungsstreulicht eine Behandlungswinkelintensitätsverteilung hat; und
Ausgeben eines Potenz- oder Wirksamkeitssignals,
wobei das Potenz- oder Wirksamkeitssignal eine Differenz zwischen der Behandlungswinkelintensitätsverteilung und der Kontrollwinkelintensitätsverteilung repräsentiert, und
wobei das Potenz- oder Wirksamkeitssignal durch den Kontroll-Imager und den Behandlungsimager verursacht wird.

2. Verfahren nach Anspruch 1, wobei der Schritt des Ausgebens des Potenz- oder Wirksamkeitssignals aufweist:
Verursachen eines Kontrollsignals durch den Kontroll-Imager,
wobei das Kontrollsignal die Kontrollwinkelintensitätsverteilung repräsentiert;
Verursachen eines Behandlungssignals durch den Behandlungsimager,
wobei das Behandlungssignal die Behandlungswinkelintensitätsverteilung repräsentiert;
Verwenden des Kontrollsignals und des Behandlungssignals, um eine Ausgabe des Potenz- oder Wirksamkeitssignals zu verursachen.

3. Verfahren nach Anspruch 2, wobei der Schritt eines Ausgebens des Potenz- oder Wirksamkeitssignals aufweist:
Berechnen eines Nettosignals;
wobei das Nettosignal eine Differenz zwischen dem Kontrollsignal und dem Behandlungssignal repräsentiert; und
Verwenden des Nettosignals, um eine Ausgabe des Potenz- oder Wirksamkeitssignals zu verursachen.

4. Verfahren nach Anspruch 3, umfassend den Schritt eines Verursachens eines historischen Signals, wobei das historische Signal eine Differenz zwischen historischen Daten und einem Element aus der Signalgruppe repräsentiert, die aus dem Behandlungssignal, dem Kontrollsignal und dem Nettosignal besteht.

5. Verfahren nach einem vorhergehenden Anspruch, wobei das Verfahren ein Verfahren eines Untersuchens der Effekte einer Behandlung eines inneren Teils einer Zelle ist.

6. Verfahren nach einem vorhergehenden Anspruch, wobei das Verfahren ein Verfahren eines Untersuchens der Effekte einer Behandlung eines äußeren Teils einer Zelle ist.

7. Abbildungsverfahren zum Untersuchen des Effekts einer Behandlung für HIV, wobei das Verfahren aufweist:
Vorbereiten einer Probe von Zellen;
Vorbereiten einer Kontrollprobe von Zellen,
wobei die Kontrollprobe von der Probe stammt;
Vorbereiten einer Behandlungsprobe von Zellen,
wobei die Behandlungsprobe von der Probe stammt, wobei eine Behandlung hinzugefügt ist;
Beleuchten oder Bestrahlen der Kontrollprobe mit einem Kontrolllicht;
Beleuchten oder Bestrahlen der Behandlungsprobe mit einem Behandlungslicht;
Detektieren eines Kontrollstreulichts zwischen 0 und 4 Grad mit einem Kontroll-Imager,
wobei das Kontrollstreulicht ein Kontrollanteil des Kontrolllichts ist, das durch die Kontrollprobe gestreut wird und durch den Kontroll-Imager zwischen 0 und 4 Grad detektiert wird,
wobei das zwischen 0 und 4 Grad detektierte Kontrollstreulicht eine Kontrollwinkelintensitätsverteilung hat;
Detektieren eines Behandlungsstreulichts zwischen 0 und 4 Grad mit einem Behandlungsimager,
wobei das Behandlungsstreulicht ein Behandlungsanteil des Behandlungslichts ist, das durch die Behandlungsprobe gestreut wird und durch den Behandlungsimager zwischen 0 und 4 Grad detektiert wird,
wobei das zwischen 0 und 4 Grad detektierte Behandlungsstreulicht eine Behandlungswinkelintensitätsverteilung hat;
Ausgeben eines Potenz- oder Wirksamkeitssignals,
wobei das Potenz- oder Wirksamkeitssignal eine Differenz zwischen der Behandlungswinkelintensitätsverteilung und der Kontrollwinkelintensitätsverteilung repräsentiert, und
wobei das Potenz- oder Wirksamkeitssignal durch den Kontroll-Imager und den Behandlungsimager durch ein Verfahren verursacht wird, das aufweist:
Verursachen eines Kontrollsignals durch den Kontroll-Imager,
wobei das Kontrollsignal die Kontrollwinkelwinkelintensitätsverteilung repräsentiert;
Verursachen eines Behandlungssignals durch den Behandlungsimager,
wobei das Behandlungssignal die Behandlungswinkelintensitätsverteilung repräsentiert;
Berechnen eines Nettosignals,
wobei das Nettosignal eine Differenz zwischen dem Kontrollsignal und dem Behandlungssignal repräsentiert und
Verwenden des Nettosignals, um eine Ausgabe des Potenz- oder Wirksamkeitssignals zu verursachen.

8. Verfahren nach Anspruch 7, umfassend den Schritt eines Verursachens eines historischen Signals, wobei das historische Signal eine Differenz zwischen historischen Daten und einem Element von der Signalgruppe repräsentiert, die aus dem Behandlungssignal, dem Kontrollsignal und dem Nettosignal besteht.

## Revendications

1. Méthode pour étudier l'effet d'un traitement pour le VIH, la méthode comprenant :
préparation d'un échantillon de cellules ;
préparation d'un échantillon de cellules de contrôle,
l'échantillon de contrôle provenant de l'échantillon ;
préparation d'un échantillon de cellules de traitement,
l'échantillon de traitement provenant de l'échantillon avec un traitement ajouté ;
éclairage de l'échantillon de contrôle avec une lumière de contrôle ;
éclairage de l'échantillon de traitement avec une lumière de traitement ;
détection de la lumière diffusée de contrôle entre 0 et 4 degrés avec un imageur de contrôle,
la lumière diffusée de contrôle étant une portion de contrôle de la lumière de contrôle qui est diffusée par l'échantillon de contrôle et détectée par l'imageur de contrôle entre 0 et 4 degrés,
la lumière diffusée de contrôle détectée entre 0 et 4 degrés ayant une répartition angulaire de l'intensité de contrôle ;
détection de la lumière diffusée de traitement entre 0 et 4 degrés avec un imageur de traitement,
la lumière diffusée de traitement étant une portion de traitement de la lumière de traitement qui est diffusée par l'échantillon de traitement et détectée par l'imageur de traitement entre 0 et 4 degrés,
la lumière diffusée de traitement détectée entre 0 et 4 degrés ayant une répartition angulaire de l'intensité de traitement ; et émission d'un signal de puissance,
le signal de puissance représentant la différence entre la répartition angulaire de l'intensité de traitement et la répartition angulaire de l'intensité de contrôle, et
le signal de puissance étant produit par l'imageur de contrôle et l'imageur de traitement.

2. Méthode selon la revendication 1, dans laquelle l'étape d'émission du signal de puissance comprend :
production d'un signal de contrôle par l'imageur de contrôle,
le signal de contrôle représentant la répartition angulaire de l'intensité de contrôle ;
production d'un signal de traitement par l'imageur de traitement,
le signal de traitement représentant la répartition angulaire de l'intensité de traitement ;
utilisation du signal de contrôle et du signal de traitement pour émettre le signal de puissance.

3. Méthode selon la revendication 2, dans laquelle l'étape d'émission du signal de puissance comprend :
calcul d'un signal net ;
le signal net représentant la différence entre le signal de contrôle et le signal de traitement ; et
utilisation du signal net pour émettre le signal de puissance.

4. Méthode selon la revendication 3, comprenant l'étape de production d'un signal historique, le signal historique représentant la différence entre des données historiques et un membre appartenant au groupe de signaux constitué par le signal de traitement, le signal de contrôle et le signal net.

5. Méthode selon l'une des précédentes revendications, dans laquelle la méthode est une méthode pour étudier l'effet d'un traitement sur une partie intérieure d'une cellule.

6. Méthode selon l'une des précédentes revendications, dans laquelle la méthode est une méthode pour étudier l'effet d'un traitement sur une partie extérieure d'une cellule.

7. Méthode d'imagerie pour étudier l'effet d'un traitement pour le VIH, la méthode comprenant :
préparation d'un échantillon de cellules ;
préparation d'un échantillon de cellules de contrôle,
l'échantillon de contrôle provenant de l'échantillon ;
préparation d'un échantillon de cellules de traitement,
l'échantillon de traitement provenant de l'échantillon avec un traitement ajouté ;
éclairage de l'échantillon de contrôle avec une lumière de contrôle ;
éclairage de l'échantillon de traitement avec une lumière de traitement ;
détection de la lumière diffusée de contrôle entre 0 et 4 degrés avec un imageur de contrôle,
la lumière diffusée de contrôle étant une portion de contrôle de la lumière de contrôle qui est diffusée par l'échantillon de contrôle et détectée par l'imageur de contrôle entre 0 et 4 degrés,
la lumière diffusée de contrôle détectée entre 0 et 4 degrés ayant une répartition angulaire de l'intensité de contrôle ;
détection de la lumière diffusée de traitement entre 0 et 4 degrés avec un imageur de traitement,
la lumière diffusée de traitement étant une portion de traitement de la lumière de traitement qui est diffusée par l'échantillon de traitement et détectée par l'imageur de traitement entre 0 et 4 degrés,
la lumière diffusée de traitement détectée entre 0 et 4 degrés ayant une répartition angulaire de l'intensité de traitement ; et émission d'un signal de puissance,
le signal de puissance représentant la différence entre la répartition angulaire de l'intensité de traitement et la répartition angulaire de l'intensité de contrôle, et
le signal de puissance étant produit par l'imageur de contrôle et l'imageur de traitement par une méthode comprenant :
production d'un signal de contrôle par l'imageur de contrôle,
le signal de contrôle représentant une répartition angulaire de l'intensité de contrôle ;
production d'un signal de traitement par un imageur de traitement,
le signal de traitement représentant la répartition angulaire de l'intensité de traitement ;
calcul d'un signal net,
le signal net représentant la différence entre le signal de contrôle et le signal de traitement, et
utilisation du signal net pour produire l'émission du signal de puissance.

8. Méthode selon la revendication 7, comprenant une étape de production d'un signal historique, le signal historique représentant la différence entre des données historiques et un membre appartenant au groupe de signaux constitué par le signal de traitement, le signal de contrôle et le signal net.
